(19) Europäisches Patentamt
European Patent Office
Office européen des brevets

(11) **EP 3 110 322 B1**

(12) **FASCICULE DE BREVET EUROPEEN**

(45) Date de publication et mention de la délivrance du brevet:
**07.06.2023 Bulletin 2023/23**

(21) Numéro de dépôt: **15709292.5**

(22) Date de dépôt: **20.02.2015**

(51) Classification Internationale des Brevets (IPC):
**A61B 5/0245** *(2006.01)* **A61B 5/327** *(2021.01)*
**A61B 5/352** *(2021.01)* **A61B 5/364** *(2021.01)*
**A61B 5/00** *(2006.01)*

(52) Classification Coopérative des Brevets (CPC):
**A61B 5/7221; A61B 5/02455; A61B 5/327;
A61B 5/352; A61B 5/364**

(86) Numéro de dépôt international:
**PCT/FR2015/050418**

(87) Numéro de publication internationale:
**WO 2015/128568 (03.09.2015 Gazette 2015/35)**

(54) **PROCÉDÉ ET DISPOSITIF DE CONTRÔLE AUTOMATIQUE DE LA QUALITÉ D'UNE SERIE RR OBTENUE À PARTIR D'UN SIGNAL CARDIAQUE**

VERFAHREN UND VORRICHTUNG ZUR AUTOMATISCHEN ÜBERPRÜFUNG DER QUALITÄT EINER AUS EINEM HERZSIGNAL ERMITTELTEN RR-INTERVALLSERIE

METHOD AND DEVICE FOR AUTOMATICALLY CHECKING THE QUALITY OF AN RR SERIES OBTAINED FROM A CARDIAC SIGNAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.02.2014 FR 1451488**

(43) Date de publication de la demande:
**04.01.2017 Bulletin 2017/01**

(73) Titulaire: **Centre Hospitalier Régional Universitaire de Lille
59037 Lille Cedex (FR)**

(72) Inventeurs:
• **LOGIER, Régis**
  **F-59520 Marquette lez Lille (FR)**
• **DE JONCKHEERE, Julien**
  **F-59160 Lomme (FR)**
• **JEANNE, Mathieu**
  **F-59800 Lille (FR)**

(74) Mandataire: **Matkowska, Franck
Matkowska & Associés
9, rue Jacques Prévert
59650 Villeneuve d'Ascq (FR)**

(56) Documents cités:
**WO-A1-2013/143893    US-A1- 2011 270 346**

• **DE JONCKHEERE J ET AL: "Heart rate variability analysis for newborn infants prolonged pain assessment", ENGINEERING IN MEDICINE AND BIOLOGY SOCIETY,EMBC, 2011 ANNUAL INTERNATIONAL CONFERENCE OF THE IEEE, IEEE, 30 août 2011 (2011-08-30), pages 7747-7750, XP032320474, DOI: 10.1109/IEMBS.2011.6091909 ISBN: 978-1-4244-4121-1**
• **VARADARAJAN V ET AL: "Assessing QT-RR interval hysteresis in 12-lead electrocardiograms", COMPUTERS IN CARDIOLOGY, 2009, IEEE, PISCATAWAY, NJ, USA, 13 septembre 2009 (2009-09-13), pages 273-276, XP031656321, ISBN: 978-1-4244-7281-9**
• **SMITAL L ET AL: "Adaptive Wavelet Wiener Filtering of ECG Signals", IEEE TRANSACTIONS ON BIOMEDICAL ENGINEERING, IEEE SERVICE CENTER, PISCATAWAY, NJ, USA, vol. 60, no. 2, 1 février 2013 (2013-02-01), pages 437-445, XP011514870, ISSN: 0018-9294, DOI: 10.1109/TBME.2012.2228482**

• VAN LAAR J O E H ET AL: "Normalized spectral power of fetal heart rate variability is associated with fetal scalp blood pH", EARLY HUMAN DEVELOPMENT, SHANNON, IR, vol. 87, no. 4, 18 janvier 2011 (2011-01-18), pages 259-263, XP028370433, ISSN: 0378-3782, DOI: 10.1016/J.EARLHUMDEV.2011.01.028 [extrait le 2011-01-21]

**Description**

**Domaine de l'invention**

La présente invention concerne le domaine du traitement numérique d'un signal bioélectrique, qui est caractéristique du rythme cardiaque d'un être vivant, et qui est désigné dans le présent texte par les termes signal cardiaque. Il s'agit par exemple, mais de manière non exclusive, d'un signal électrocardiographique (ECG). Dans ce domaine technique, l'invention concerne le contrôle automatique de la qualité d'une série RR, obtenue par échantillonnage d'un signal cardiaque

**Art antérieur**

D'un point de vue physiologique, le coeur d'un être vivant, isolé de toute influence extérieure, se contracte automatiquement de façon très régulière comme un métronome, sous l'action du noeud sinusal qui génère un influx nerveux indépendant, et par là-même provoque une contraction spontanée du muscle cardiaque. Le coeur n'est toutefois pas isolé, mais est relié au Système Nerveux Autonome (SNA), par l'intermédiaire des systèmes parasympathique et sympathique. Ce système nerveux autonome influe sur l'activité du coeur : le système sympathique accélère le rythme cardiaque, tandis que le système parasympathique le ralentit. Ainsi, malgré une certaine autonomie, le coeur subit des influences du système nerveux autonome, ce qui permet notamment à l'organisme d'un être vivant d'adapter le rythme cardiaque en fonction de ses besoins, dans des limites toutefois raisonnables. On comprend en conséquence que l'analyse de l'évolution dans le temps du rythme cardiaque, et en particulier des variations du rythme cardiaque (variation des battements du coeur), permet d'obtenir une information importante sur l'activité du système cardiaque, et plus particulièrement sur l'activité du système nerveux autonome. Or la connaissance de l'activité du SNA peut être d'une aide précieuse dans l'élaboration d'un diagnostic de bon nombre de situations cliniques. Sur ce sujet, on pourra se référer par exemple à la publication ci-après : Lacroix D, Logier R., Kacet S., Hazard J-R, Dagano J. (1992) : « Effects of consecutive administration of central and peripheral anticholinergic agents on respiratory sinu arrhytmia in normal subjects, J. of the Autonomie Nervous System », Vol 39, pages 211-218.

Pour étudier ces fluctuations du rythme cardiaque, on a déjà depuis 1970 développé différentes techniques de filtrage et d'analyse spectrale d'un signal qui représente l'évolution dans le temps du rythme (ou fréquence) cardiaque instantané, et qui est obtenu après échantillonnage d'un signal bioélectrique analogique, caractéristique du rythme cardiaque d'un être vivant, et dit par la suite « signal cardiaque analogique ».

Pour acquérir ce signal cardiaque, différentes techniques d'acquisition invasives ou non invasives sont connues. Une technique invasive connue consiste par exemple à utiliser un capteur de pression sanglante relié à un cathéter introduit dans une artère. Parmi les méthodes non invasives connues, on trouve par exemple l'utilisation d'un capteur de pouls infrarouge, ou l'acquisition d'un signal électrocardiographique (ECG) au moyen d'un électrocardiographe. Cette dernière méthode d'acquisition d'un signal ECG est en pratique la plus couramment utilisée à ce jour, car outre son caractère non invasif, elle permet avantageusement d'obtenir un signal plus précis que celui obtenu par exemple au moyen d'un capteur de pouls infrarouge.

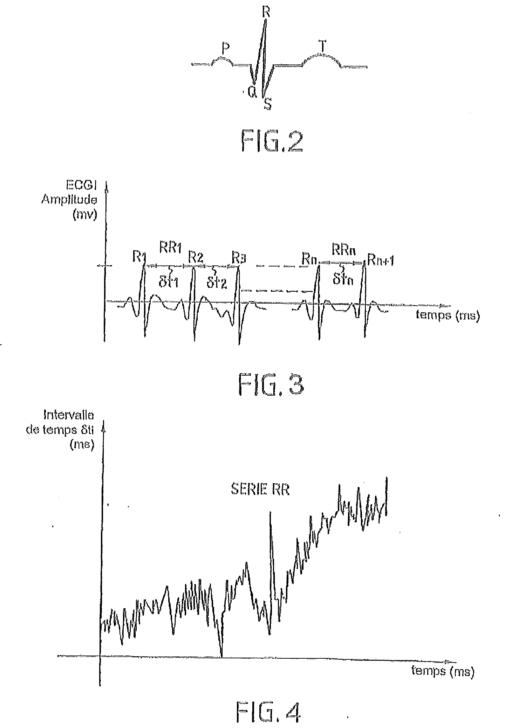

Le signal ECG est de manière connue constitué d'une succession de dépolarisations électriques dont l'allure est représentée sur la figure 3 annexée. L'onde P, qui correspond à la dépolarisation des oreillettes, présente une faible amplitude, et une forme de dôme. L'espace PQ traduit le temps de conduction auriculo-ventriculaire. Le complexe QRS reflète la contraction ventriculaire, et l'onde T la repolarisation ventriculaire. En pratique, on considère le pic R comme marqueur de la systole ventriculaire, c'est-à-dire du battement cardiaque.

En pratique, l'onde R étant le plus souvent la partie la plus fine et la plus ample du QRS, elle est généralement utilisée pour localiser ponctuellement le battement cardiaque avec une très bonne précision, en pratique de l'ordre du millième de seconde. Ainsi l'intervalle de temps entre deux ondes R successives caractérise de manière précise le temps séparant deux battements cardiaques successifs ; c'est la période du signal ECG, et l'inverse de cette période donne la fréquence cardiaque instantanée.

Pour construire automatiquement le signal, dit par la suite série RR, représentant l'évolution dans le temps du rythme cardiaque instantané, on échantillonne le signal ECG qui est un signal analogique (conversion analogique/numérique du signal ECG), et on traite le signal ECG numérique échantillonné, en détectant automatiquement les ondes R dans ce signal numérique. Une série RR est ainsi de manière usuelle, constituée d'une pluralité d'échantillons (ou points) $RR_i$ successifs, chaque échantillon $RR_i$ étant fonction de l'intervalle de temps séparant deux ondes R successives du signal ECG.

Il faut toutefois souligner d'une part que l'on peut également utiliser les autres ondes de dépolarisation (P, Q, S ou T) du signal ECG pour caractériser la fréquence cardiaque, même si la précision de la mesure est moins bonne qu'en utilisant les ondes R. D'autre part, en fonction de la technique d'acquisition choisie, le signal cardiaque peut présenter une forme différente de celle précitée d'un signal ECG. Ce signal cardiaque n'est pas nécessairement analogique, mais peut être un signal numérique. En conséquence, dans le présent texte, le terme série RR ne se limite pas à la définition particulière précitée basée sur les ondes R d'un signal ECG, mais se définit d'une manière plus générale dans le cadre de la présente invention comme une série de plusieurs échantillons numériques dits $RR_i$, obtenue à partir d'un signal cardiaque qui est caractéristique du rythme cardiaque, chaque échantillon $RR_i$ étant fonction de l'intervalle de temps entre deux battements cardiaques successifs. Chaque échantillon $RR_i$ peut être proportionnel, et notamment égal, à l'intervalle de temps entre deux battements cardiaques successifs ou inversement proportionnel à l'intervalle de temps entre deux battements cardiaques successifs.

En pratique, les perturbations dans le signal cardiaque, et en particulier dans un signal ECG, induisent, dans la série RR issue de ce signal cardiaque, des variations brutales de faible durée couramment appelées artefacts.

Les perturbations, à l'origine d'artefacts dans la série RR, peuvent être physiologiques et liées intrinsèquement à un dysfonctionnement momentané du système cardiaque ; il s'agit par exemple d'une extrasystole. Ces perturbations peuvent également être extérieures et non liées au fonctionnement du système cardiaque ; il s'agit par exemple d'un mouvement du patient altérant brièvement le signal de mesure.

Les artefacts dans une série RR peuvent se traduire par un unique échantillon erroné ou par une pluralité d'échantillons successifs erronés. En pratique, un artefact dans la série RR peut être assimilé à une impulsion de Dirac, et se traduit dans le domaine fréquentiel par un spectre continu rectangulaire à large bande. Par conséquent, dans l'hypothèse où on transposerait dans le domaine fréquentiel (par transformée de Fourier ou autre) une série RR, sans prendre au préalable de précautions particulières, la présence d'artefacts dans la sérié RR se traduirait dans le domaine fréquentiel par l'obtention d'un spectre fréquentiel de la série RR très perturbé, de forme rectangulaire de large bande, masquant le spectre du signal réel.

Pour cette raison, pour obtenir une information fréquentielle correcte, il est primordial d'éliminer les artefacts avant de réaliser la transposition en fréquence.

On a ainsi proposé dans la demande de brevet internationale WO 02/069178, ainsi que dans l'article Logier R, De Jonckheere J, Dassonneville A. , « An efficient algorithm for R-R intervals series filtering ». Conf Proc IEEE Eng Med Biol Soc. 2004;6:3937-40, des algorithmes de filtrage numérique, qui d'une manière générale permettent de filtrer en temps réel une série RR, obtenue à partir d'un signal cardiaque, en détectant automatiquement dans la série RR la présence d'un ou plusieurs d'échantillons $RR_i$ successifs erronés, et en remplaçant automatiquement dans la série RR les échantillons $RR_i$ erronés qui ont été détectés par des échantillons $RR_c$ corrigés. La détection d'échantillons $RR_i$ erronés peut être réalisée de différentes manières et les échantillons ($RR_c$) corrigés peuvent également être calculés de diverses manières, et par exemple, mais non exclusivement par interpolation linéaire.

L'article par Varadarajan V et al.: "Assessing QT-RR interval hystérésis in 12-lead electrocardiograms",COMPUTERS IN CARDIOLOGY, 2009, IEEE, PISCATAWAY, NJ, USA, pages 273-276, publié le 13 septembre 2009, décrit le calcul d'une série d'intervalles QT à partir d'une série d'intervalles RR. La norme L2 de la partie des intervalles QT non corrélée aux intervalles RR est utilisée pour déterminer la qualité du signal d'une dérivation ECG.

Un problème de ces algorithmes de filtrage, désignés par la suite algorithmes ou procédé de filtrage « *avec reconstruction des échantillons erronés d'une série RR* » réside dans le fait que la reconstruction de la série RR, par remplacement des échantillons $RR_i$ erronés qui ont été détectés par des échantillons RRc corrigés, peut aboutir en final à une série RR en partie reconstruite qui est elle-même partiellement ou totalement faussée, en particulier lorsque le signal cardiaque qui a été prélevé est de mauvaise qualité. Le défaut de qualité de ce signal cardiaque peut découler de nombreux facteurs, tel que par exemple, et de manière non limitative et non exhaustive, un mauvais positionnement des électrodes ou capteurs de mesure du signal cardiaque, une amplification insuffisante du signal dans la chaine de traitement de signal, etc...

Or la reconstruction d'une série RR faussée n'est à ce jour pas détectée par les algorithmes de filtrage d'une série RR. Il en résulte que l'information fournie par ces algorithmes de filtrage peut être totalement erronée ou non significative sans que l'on s'en aperçoive.

**Objectif de l'invention**

La présente invention vise à proposer une solution de contrôle automatique de la qualité d'une série RR obtenue par à partir d'un signal cardiaque.

**Résumé de l'invention**

L'invention a ainsi pour premier objet un procédé de contrôle de la qualité d'une série RR initiale constituée d'une pluralité d'échantillons (RRi) qui sont fonction respectivement des intervalles de temps ($\delta$ti) qui séparent deux battements cardiaques successifs, procédé mis en oeuvre par des moyens électroniques de traitement et comportant une étape de ré-échantillonnage de la série RR de manière à obtenir une série RR ré-échantillonnée. De manière caractéristique selon l'invention, ledit procédé comporte en outre une étape de contrôle automatique de la qualité de la série RR, en calculant automatiquement au moins la norme mathématique, dans une fenêtre glissante, de la série RR ré-échantillonnée, ladite norme mathématique étant donnée par la formule suivante :

$$NORME = \sqrt{\sum_{i=1}^{N}\left( RR_i - \frac{1}{N}\sum_{i=1}^{N}\left( RR_i \right) \right)^2}$$

où N est le nombre d'échantillons $RR_i$ dans ladite fenêtre.

Dans le présent texte, et notamment dans les revendications, on désigne par les termes « signal cardiaque », tout signal physique caractéristique du rythme (ou de la fréquence) cardiaque instantanée de l'être vivant. Pour la mise en oeuvre de l'invention, différentes techniques invasives ou non invasives peuvent être utilisées pour acquérir ce signal cardiaque. Une technique invasive connue consiste par exemple à utiliser un capteur de pression sanglante relié à un cathéter introduit dans une artère. Parmi les méthodes non invasives connues (et pour lesquelles on optera de préférence), on peut citer par exemple l'utilisation d'un capteur de pouls infrarouge, l'utilisation d'un capteur à ultrasons permettant la détection des cycles cardiaques, du type du capteur mis en oeuvre dans un cardiotocographe, ou encore l'acquisition d'un signal électrocardiographique (ECG). L'acquisition d'un signal électrocardiographique (ECG) est en pratique la méthode la plus couramment utilisée, car outre son caractère non invasif, elle permet d'obtenir un signal cardiaque plus précis que celui obtenu par exemple au moyen d'un capteur de pouls infrarouge.

Dans le présent texte, et notamment dans les revendications, on désigne d'une manière générale par les termes « série RR », une série de plusieurs échantillons successifs $RR_i$, obtenus à partir d'un signal cardiaque caractéristique du rythme cardiaque de l'être vivant, chaque échantillon RRi étant d'une manière générale fonction d'un intervalle de temps (δti) entre deux battements cardiaques successifs. Généralement, chaque échantillon (RRi) est proportionnel, et plus particulièrement égal, à l'intervalle de temps (δti) entre deux battements cardiaques successifs. Chaque échantillon (RRi) peut également être proportionnel, et plus particulièrement égal, à l'inverse (1/δti) de l'intervalle de temps entre deux battements cardiaques successifs.

Dans l'exemple préféré de réalisation décrit ci-après en référence aux figures annexées, cette série RR est plus particulièrement construite à partir des ondes R d'un signal ECG. Ceci n'est toutefois pas limitatif de l'invention. Dans le cas d'un signal cardiaque type ECG, on peut construire la série dite « RR » en utilisant les autres ondes de dépolarisation (P, Q, S ou T) du signal ECG pour construire la série RR, la précision étant toutefois moins bonne qu'en utilisant les ondes R du signal ECG. Egalement, lorsque le signal cardiaque n'est pas un signal ECG, les échantillons de la série RR ne sont pas calculés en déterminant l'intervalle de temps (δti) séparant deux ondes R successives du signal ECG, mais sont d'une manière plus générale déterminés en détectant dans le signal cardiaque l'intervalle de temps entre deux battements cardiaques successifs.

L'invention a également pour objet un dispositif de traitement d'une série RR constituée d'une pluralité d'échantillons (RRi) qui sont fonction respectivement des intervalles de temps (δti) qui séparent deux battements cardiaques successifs, ledit dispositif étant conçu pour contrôler automatiquement la qualité de la série RR en mettant en oeuvre le procédé susvisé.

L'invention a pour autre objet un système d'acquisition et de traitement d'un signal cardiaque, ledit système comportant des moyens électroniques d'acquisition d'un signal cardiaque, et des moyens électroniques de traitement conçus pour construire une série RR initiale, à partir du signal cardiaque acquis par les moyens électroniques d'acquisition, ladite série RR étant constituée d'une pluralité d'échantillons ($RR_i$) qui sont fonction respectivement des intervalles de temps (δti) qui séparent deux battements cardiaques successifs du signal cardiaque. De manière caractéristique selon l'invention lesdits moyens électroniques de traitement sont conçus pour contrôler automatiquement la qualité de série RR en mettant en oeuvre le procédé susvisé.

L'invention a également pour objet un programme informatique comprenant un moyen de code de programme informatique apte à être exécuté par des moyens électroniques de traitement, et permettant, lorsqu'il est exécuté par des moyens électroniques de traitement, de mettre en oeuvre le procédé susvisé de contrôle de la qualité d'une série RR.

## Brève description des figures

D'autres caractéristiques et avantages de l'invention apparaîtront plus clairement à la lecture de la description détaillée ci-après d'un exemple préféré de réalisation du procédé de l'invention, laquelle description détaillée est donnée à titre d'exemple non limitatif et non exhaustif, et en référence aux dessins annexés sur lesquels :

- la figure 1 représente de manière schématique les principaux éléments d'un exemple de système d'acquisition et de traitement d'un signal ECG mettant en oeuvre le procédé de l'invention,
- la figure 2 représente l'ensemble d'ondes (PQRST) caractéristique d'un battement cardiaque dans un signal ECG,

- la figure 3 représente un exemple de signal numérique ECG, obtenu après échantillonnage d'un signal ECG analogique,
- la figure 4 représente un exemple de série RR (encore désigné signal RR) construite à partir du signal de la figure 3.

**Description détaillée**

Système d'acquisition et de traitement du signal cardiaque

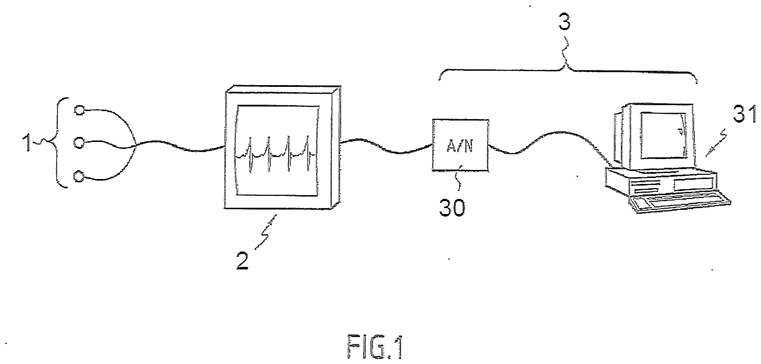

On a représenté sur la figure 1 un exemple de système d'acquisition et de traitement du signal cardiaque d'un être vivant (humain ou animal) qui est utilisé pour la mise en oeuvre du procédé de l'invention.
Ce système comporte :

- des moyens électroniques usuels d'acquisition d'un signal ECG, comprenant plusieurs électrodes de mesure 1 reliées en entrée à un moniteur électrocardiographique (ECG) 2,
- des moyens électroniques 3 de traitement du signal ECG délivré en sortie par le moniteur ECG 2.

Les moyens de traitement 3 du signal ECG comprennent un convertisseur analogique/numérique 30, et une unité de traitement électronique 31. L'entrée du convertisseur 30 est reliée à la sortie du moniteur ECG 2, et la sortie du convertisseur 30 est reliée à un port d'entrée de l'unité de traitement électronique 31. Dans un exemple particulier de réalisation, non limitatif de l'invention, l'unité de traitement 31 est constituée par un micro-ordinateur, le convertisseur 30 étant relié à un port série RS232 de ce micro-ordinateur. L'invention n'est pas limitée à la mise en oeuvre d'un micro-ordinateur, l'unité de traitement électronique 31 pouvant être implémentée de manière différente, et par exemple sous la forme d'un circuit électronique programmable de type FPGA ou sous la forme d'un circuit intégré de type ASIC.
En fonctionnement, les électrodes 1 sont appliquées sur le corps de l'être vivant, et le moniteur ECG 2 délivre en sortie de manière usuelle un signal électrique analogique, dit signal ECG, qui pour chaque battement cardiaque, a la forme du signal représenté à la figure 2.
En référence à la figure 2, pour chaque battement cardiaque, ce signal électrocardiographique (ECG) est constitué d'un ensemble d'ondes électriques :

- l'onde P, qui correspond à la dépolarisation des oreillettes, et qui présente une faible amplitude et une forme de dôme ;
- l'espace PQ qui traduit le temps de conduction auriculo-ventriculaire;
- l'onde R considérée en pratique comme marqueur de la systole ventriculaire, ou du battement cardiaque, le complexe QRS reflétant la contraction ventriculaire, et
- l'onde T qui reflète la repolarisation ventriculaire.

Ce signal ECG analogique est numérisé par le convertisseur 4, avec une fréquence d'échantillonnage (fc) prédéterminée, valant par exemple 256 Hz.
Le signal échantillonné délivré en sortie du convertisseur 30 (signal représenté sur la figure 3) est traité par l'unité de traitement 31, au moyen d'un logiciel de traitement spécifique (logiciel de filtrage) qui est décrit en détail ultérieurement. Ce logiciel de filtrage est stocké en mémoire de l'unité de traitement 31, et permet, lorsqu'il est exécuté, de construire automatiquement, à partir du signal numérique délivré par le convertisseur analogique/numérique 30, une série RR avec le cas échéant reconstruction automatique des échantillons erronés $RR_i$, et de calculer automatiquement un indice de qualité NivQual qui permet de contrôler la qualité de la série RR le cas échéant en partie reconstruite.
Une variante préférée de ce logiciel de filtrage va à présent être détaillée.

Exemple d'algorithme du logiciel de filtrage

Dans une variante particulière de réalisation de l'invention, les principales étapes successives de l'algorithme de filtrage sont les suivantes :

1. Acquisition et construction des échantillons $RR_i$ à partir du signal délivré par le convertisseur analogique/numérique 30,
2. Filtrage de la série RR avec le cas échéant détection automatique des échantillons $RR_i$ erronés et remplacement par des échantillons reconstruits identifiés dans la suite échantillons RRc.
3. Ré-échantillonnage de la série RR à une fréquence f prédéfinie pour obtenir des échantillons $RR_i$ ré-échantillonnés
4. Sélection des échantillons $RR_i$ compris dans une fenêtre temporelle de n secondes (n>1/f)
5. Calcul d'un indice de qualité NivQual
6. Décalage, d'un pas temporel valant p secondes (de préférence $p \le n$), de la fenêtre temporelle de n secondes,

6

et réitération du calcul à partir de l'étape 2. Ce décalage correspond au glissement de la fenêtre temporelle de sélection des échantillons.

En pratique, le système peut être programmé pour être utilisé en temps réel ou en temps différé.

Lorsque le système est utilisé en temps différé, on réalise dans un premier temps l'étape 1 en temps réel en sorte de construire tous les échantillons $RR_i$ sur toute la période d'analyse souhaitée ; l'intégralité de ces échantillons $RR_i$ successifs est stockée en mémoire, par exemple dans un fichier d'acquisition en mémoire de l'unité de traitement 31. Dans un second temps, les étapes 2 à 6 sont effectuées en boucle, en différé, sur les échantillons $RR_i$ stockés dans le fichier d'acquisition.

Lorsque le système fonctionne en temps réel, l'étape 1 de construction des échantillons $RR_i$ d'une part, et les autres étapes de traitement 2 à 6 d'autre part, sont exécutées par deux modules logiciels distincts fonctionnant en parallèle, le premier module de construction (étape 1) alimentant le second module de traitement et de calcul (étapes 2 à 6) par exemple par l'intermédiaire d'un fichier ou registre tampon ou équivalent.

Les étapes 1 à 5 vont présent être détaillées.

Etape 1 : Acquisition et construction des échantillons $RR_i$

L'acquisition et la construction des échantillons RRi est réalisée par un premier sous-module logiciel qui est alimenté en entrée avec les données numériques successives constitutives du signal ECG numérisé (signal de la figure 3) délivré par le convertisseur analogique numérique 30. Chaque donnée (ou point) du signal ECG est définie par l'amplitude instantanée $ECG_i$ du signal ECG, et par l'instant $t_i$ d'échantillonnage ($t_i = n_i/fc$, avec $n_i$ numéro d'échantillon et fc représentant la fréquence d'échantillonnage du convertisseur 30).

Le premier sous-module d'acquisition des échantillons $RR_i$ est conçu pour détecter automatiquement chaque pic $R_i$ successif dans le signal numérique délivré par le convertisseur 30, et pour construire automatiquement une série RR (figure 4) constituée d'une succession de d'échantillons $RR_i$. Chaque échantillon $RR_i$ est défini par le couple de coordonnées : ti [un instant (ou numéro) d'échantillonnage] ; intervalle de temps δti (exprimé en multiple de la fréquence d'échantillonnage fc) séparant un pic $R_i$ du pic suivant $R_{i+1}$ (dans une autre variante il pourrait s'agir du pic précédent $R_{i-1}$).

De manière usuelle en soi, l'onde R étant le plus souvent la partie la plus fine et la plus ample du QRS, elle est de préférence utilisée pour détecter le battement cardiaque avec une très bonne précision, l'intervalle de temps δti correspondant en pratique au temps séparant deux battements cardiaques successifs. Néanmoins, dans une autre variante, on pourrait envisager d'utiliser d'autres ondes (par exemple onde Q ou onde S) d'un battement cardiaque du signal ECG pour détecter et construire la série RR. Dans une autre variante, on pourrait également envisager d'utiliser d'autres signaux cardiaques comme l'onde pléthysmographique ou la pression artérielle invasive.

Etape 2 : Filtrage de la série RR avec le cas échéant détection automatique des échantillons $RR_i$ erronés et remplacement par des échantillons reconstruits RRc.

Cette étape de filtrage consiste d'une manière générale à détecter automatiquement dans la série RR la présence d'un ou plusieurs d'échantillons $RR_i$ successifs erronés, et à remplacer automatiquement dans la série RR les échantillons $RR_i$ erronés qui ont été détectés par des échantillons reconstruits RRc. Le nombre d'échantillons reconstruits RRc est la plupart du temps différent du nombre d'échantillons erronés qui ont été détectés.

Cette étape de filtrage avec reconstruction automatique des échantillons $RR_i$ erronés est connue en soi, et des exemples de mise en oeuvre de cette étape de filtrage sont par exemple décrits dans la demande de brevet internationale WO 02/069178, ainsi que dans l'article Logier R, De Jonckheere J, Dassonneville A. , « An efficient algorithm for R-R intervals series filtering ». Conf Proc IEEE Eng Med Biol Soc. 2004;6:3937-40.

Il convient néanmoins de noter que dans le cadre de l'invention, la détection d'échantillons $RR_i$ erronés n'est pas limitée aux méthodes de détection décrites dans ces deux publications susvisées, et les échantillons reconstruits $RR_c$ peuvent également être calculés de diverses manières, et par exemple, mais non exclusivement, par interpolation linéaire tel que décrit dans les deux publications susvisées.

Chaque échantillon reconstruit RRc de la série RR est identifié, par exemple par une variable d'identification associée de type drapeau. Ainsi, à l'issue de cette étape, la série RR est constituée d'échantillons $RR_i$ dont certains sont le cas échéant identifiés par leur variable d'identification comme échantillons reconstruits RRc.

Etape 3 : Ré-échantillonnage de la série RR à une fréquence f prédéfinie pour obtenir des échantillons $RR_i$ ré-échantillonnés

La série RR filtrée (figure 4) délivrée par le premier sous-module précité est ré-échantillonnée automatiquement par un second sous-module logiciel à une fréquence prédéfinie f, qui est de préférence inférieure à la fréquence d'échantillonnage fc (par exemple, pour une fréquence d'échantillonnage fc valant 250 Hz, on fixera la fréquence f de ré-échantillonnage à 8hz). L'objectif de ce ré-échantillonnage est d'obtenir en sortie une série RR dont les échantillons $RR_i$ sont équidistants d'un point de vue temporel, c'est-à-dire en d'autres termes une série RR dont les instants d'échantillonnage sont réguliers. Ce ré-échantillonnage est réalisé de manière connue en soi par interpolation, et par exemple par interpolation linéaire.

Au cours de ce ré-échantillonnage chaque échantillon reconstruit RRc est remplacé selon le cas par un ou plusieurs échantillons reconstruits ré-échantillonnés RRrc.

Chaque échantillon reconstruit et ré-échantillonné RRrc de la série RR est identifié, par exemple par une variable d'identification associée de type drapeau. Ainsi, à l'issue de cette étape, la série RR est constituée d'échantillons $RR_i$ dont certains sont le cas échéant identifiés par leur variable d'identification comme échantillons reconstruits et ré-échantillonnés RRrc. Etape 4 : Sélection des échantillons $RR_i$ (de la série RR le cas échéant en partie reconstruite et re-échantillonnée) compris dans une fenêtre temporelle principale de n secondes (n>1/f)

Cette étape revient à isoler un nombre N d'échantillons $RR_i$ successifs (N=n.f). A titre indicatif, on choisit par exemple une fenêtre principale de 64 secondes (n=64), ce qui correspond à 512 échantillons $RR_i$ successifs (N=512), pour une fréquence f de ré-échantillonnage de 8hz.

Les étapes suivantes sont appliquées aux échantillons compris dans cette fenêtre principale.

Etape 5 : Calcul d'un indice de qualité NivQual

Cette étape est réalisée au moyen d'un sous-module logiciel qui permet de calculer automatiquement un indice de qualité NivQual significatif de la qualité de la série RR.

Dans la variante particulière décrite en détail ci-après, cet indice de qualité NivQual présente deux niveaux de qualité de 0 ou 1.

Plus particulièrement, l'indice de qualité NivQual est basé sur deux variables ($FC_i$ ; NORME ) qui sont calculées à l'étape 5 :

  1/la valeur de la fréquence cardiaque instantanée ($FC_i$) calculée sur chaque échantillon $RR_i$ de la série RR issue de l'étape 2, c'est-à-dire de la série RR après filtrage (le cas échéant en partie reconstruite) et avant ré-échantillonnage.

  2/ la norme mathématique (NORME) des échantillons $RR_i$ de la série RR (le cas échéant en partie reconstruite, et ré-échantillonnée), issus de l'étape 4 de sélection dans la fenêtre temporelle de n secondes.

La fréquence cardiaque est définie par $FC_i = 60000/RR_i$ , où $RR_i$ est la valeur instantanée de l'échantillon $RR_i$ en milliseconde.

Le calcul de la norme mathématique de la série RR ré-échantillonnée à la fréquence f dans la fenêtre de n secondes consiste dans un premier temps, à calculer la valeur moyenne M des $RR_i$ dans la fenêtre.

$$M = \tfrac{1}{N} \sum_{i=1}^{N} (RR_i)$$

où RRi représente la valeur de chaque intervalle RR et N le nombre d'échantillons dans la fenêtre.

Cette valeur moyenne est alors soustraite à chaque intervalle $RR_i$ de la fenêtre.

$$RR_i = (RR_i - M).$$

Les valeurs RRi obtenues sont utilisées pour le calcul de la norme (*NORME*), soit :

$$NORME = \sqrt{\sum_{i=1}^{N} \left( RR_i - \tfrac{1}{N} \sum_{i=1}^{N} (RR_i) \right)^2}$$

Un exemple d'algorithme pour le calcul de l'indice de qualité NivQual à partir des deux variables susvisées ($FC_i$ ; NORME ) est donné ci-après :

$$\text{Si}$$

$$((\text{NORME} < \text{NormMin})$$

$$\text{ou}$$

$$(\text{NORME} > \text{NormMax})$$

$$\text{ou}$$

$$(\text{FC}_i > \text{FCMax})$$

$$\text{ou}$$

$$(\text{FC}_i < \text{FCMin}))$$

$$\text{alors Nivqual} = 0$$

$$\text{SINON Nivqual} = 1$$

Les valeurs des paramètres FCMax, FCmin, NormMax, NormMin sont des constantes prédéfinies, qui dépendent par exemple de l'âge de l'être humain ou qui dépendent par exemple de l'espèce animale dans le cadre d'une application vétérinaire. Les valeurs des seuils FCMax, FCmin sont celles habituellement utilisées par l'ensemble des dispositifs de monitoring cardiaque. Les valeurs des seuils NormMax, NormMin de la norme sont par exemple déterminées expérimentalement sur plus de 200 individus dans chaque catégorie.
A titre d'exemple non limitatif :

- pour un nouveau-né : FCMax=250 ; FCMin=80 ; Normmax=3 ; Normmin=0
- pour un adulte : FCMax=180 ; FCMin=30 ; Normmax=4 ; Normmin=0.07

L'indice de qualité NivQual calculé à chaque étape 5 peut par exemple être affiché, notamment en temps réel, de manière à informer un praticien du niveau de qualité du signal RR mesuré.
Dans le cas d'un indice de qualité NivQual égal à 0, on considère que la série RR issue de l'étape 1 est de trop mauvaise qualité, et est en réalité inexploitable. Ce défaut de qualité de la série RR peut découler de nombreux facteurs, tel que par exemple, et de manière non limitative et non exhaustive, un mauvais positionnement des électrodes 1 ou capteurs de mesure du signal cardiaque, une amplification insuffisante du signal dans la chaine de traitement de signal, etc...
En cas de calcul d'un indice de qualité NivQual égal à 0, l'unité de trainement 31 peut être programmée pour déclencher automatiquement plusieurs actions, parmi lesquelles et de manière non exhaustive, le déclenchement d'une alarme visuelle et/ou sonore et/ou la réinitialisation de l'étape 1 d'acquisition des échantillons RRi, avec notamment une modification manuelle ou automatique du gain du signal source (ECG).
Dans le cadre de l'invention, pour la mise en oeuvre de l'étape 5, l'algorithme de calcul de cet indice de qualité NivQual peut être simplifié en prenant en compte uniquement le paramètre NORME.
Dans une autre variante de réalisation, l'étape de filtrage 2 (détection et reconstruction des échantillons erronés) peut être omise. Dans ce cas, le calcul de la norme (NORME) et le calcul de la fréquence cardiaque instantanée (FCi) sont effectués dans une fenêtre glissante directement sur les échantillons de la série RR initiale issue de l'étape 1.

## Revendications

1.  Procédé de contrôle de la qualité d'une série RR initiale constituée d'une pluralité d'échantillons ($\text{RR}_i$) qui sont fonction respectivement des intervalles de temps ($\delta ti$) qui séparent deux battements cardiaques successifs, procédé mis en oeuvre par des moyens électroniques de traitement et comportant une étape de ré-échantillonnage de la série RR de manière à obtenir une série RR ré-échantillonnée, **caractérisé en ce que** ledit procédé comporte en outre une étape de contrôle automatique de la qualité de la série RR, en calculant automatiquement au moins la norme mathématique (NORME), dans une fenêtre glissante, de la série RR ré-échantillonnée, ladite norme mathématique étant donnée par la formule suivante :

$$NORME = \sqrt{\sum_{i=1}^{N} \left( RR_i - \frac{1}{N} \sum_{i=1}^{N} (RR_i) \right)^2}$$

où N est le nombre d'échantillons $RR_i$ dans ladite fenêtre.

**2.** Procédé selon la revendication 1, au cours duquel on détecte automatiquement dans la série RR initiale si un ou plusieurs échantillons (RRi) successifs sont erronés, et on corrige automatiquement dans la série RR le ou les échantillons (RRi) détectés comme étant erronés en les remplaçant par un ou plusieurs échantillons reconstruits (RRc), de manière à obtenir une série RR le cas échéant en partie reconstruite, et dans lequel le ré-échantillonnage est réalisé sur la série RR le cas échéant en partie reconstruite, de manière à obtenir une série RR le cas échéant en partie reconstruite et ré-échantillonnée.

**3.** Procédé selon l'une quelconque des revendications précédentes, dans lequel on déclenche automatiquement une action lorsque la norme mathématique (NORME) qui est calculée est inférieure à une valeur prédéfinie (NormMin), ou lorsque la norme mathématique (NORME) qui est calculée est supérieure à une valeur prédéfinie (NormMax), ou lorsque la norme mathématique qui est calculée est en dehors d'une fourchette prédéfinie (NormMin ; NormMax).

**4.** Procédé selon l'une quelconque des revendications précédentes, dans lequel on contrôle automatiquement la qualité de la série RR en calculant également la fréquence cardiaque instantanée $FC_i$, avec $FC_i = 60000/RR_i$, $RR_i$ étant la valeur instantanée en milliseconde d'un échantillon ($RR_i$) de la série RR.

**5.** Procédé selon la revendication 4, dans lequel on déclenche automatiquement une action lorsque la fréquence cardiaque instantanée FCi qui est calculée est inférieure à une valeur prédéfinie (FCMin).

**6.** Procédé selon la revendication 4, dans lequel on déclenche automatiquement une action lorsque la fréquence cardiaque instantanée FCi qui est calculée est supérieure à une valeur prédéfinie (FCMax).

**7.** Procédé selon la revendication 4, dans lequel on déclenche automatiquement une action lorsque la fréquence cardiaque instantanée FCi qui est calculée est en dehors d'une fourchette prédéfinie (FCMin ; FCMax).

**8.** Procédé selon l'une quelconque des revendications 3, 5, 6, 7, dans lequel l'action qui est déclenchée comprend le déclenchement d'une alarme visuelle et/ou sonore.

**9.** Procédé selon l'une quelconque des revendications 3, 5, 6, 7, dans lequel l'action qui est déclenchée comprend la réinitialisation de l'acquisition et de la construction des échantillons ($RR_i$) de la série RR initiale.

**10.** Procédé selon l'une quelconque des revendications précédentes, dans lequel on calcule un indice de qualité (NivQual) à partir au moins de la norme mathématique (NORME) qui est calculée.

**11.** Procédé selon l'une quelconque des revendications précédentes, dans lequel on calcule au moins un indice de qualité (NivQual) à partir de la fréquence cardiaque instantanée $FC_i$, avec $FC_i = 60000/RR_i$, $RR_i$ étant la valeur instantanée en milliseconde d'un échantillon ($RR_i$) de la série RR.

**12.** Procédé selon l'une quelconque des revendications précédentes, comprenant l'acquisition et la construction en temps réel des échantillons ($RR_i$) successifs de la série RR initiale à partir d'un signal cardiaque, et dans lequel le ré-échantillonnage et le contrôle automatique de la qualité de la série RR sont effectués en temps réel au fur et à mesure de ladite acquisition et construction des échantillons (RRi) successifs de la série RR initiale.

**13.** Dispositif (3) de traitement d'une série RR constituée d'une pluralité d'échantillons ($RR_i$) qui sont fonction respectivement des intervalles de temps ($\delta ti$) qui séparent deux battements cardiaques successifs, ledit dispositif (3) étant agencé de manière à contrôler automatiquement la qualité de la série RR en mettant en oeuvre le procédé visé à l'une quelconque des revendications précédentes.

**14.** Système d'acquisition et de traitement d'un signal cardiaque, ledit système comportant des moyens (1,2) électroniques d'acquisition d'un signal cardiaque, et des moyens électroniques de traitement (3) agencés de manière à construire une série RR initiale, à partir du signal cardiaque acquis par les moyens (1,2) électroniques d'acquisition, ladite série RR étant constituée d'une pluralité d'échantillons ($RR_i$) qui sont fonction respectivement des intervalles de temps ($\delta ti$) qui séparent deux battements cardiaques successifs du signal cardiaque, lesdits moyens électroniques de traitement (3) étant agencés de manière à contrôler automatiquement la qualité de série RR en mettant en oeuvre le procédé visé à l'une quelconque des revendications 1 à 12.

15. Programme informatique comprenant un moyen de code de programme informatique apte à être exécuté par des moyens électroniques de traitement (3), et permettant, lorsqu'il est exécuté par des moyens électroniques de traitement (3), de mettre en œuvre le procédé de contrôle de la qualité d'une série RR visé à l'une quelconque des revendications 1 à 12.

**Patentansprüche**

1. Verfahren zur Kontrolle der Qualität einer anfänglichen RR-Reihe, die aus einer Vielzahl von Abtastungen (RRi) gebildet ist, die jeweils eine Funktion von zwei aufeinanderfolgende Herzschläge trennenden Zeitintervallen ($\delta ti$) sind, wobei das Verfahren durch elektronische Verarbeitungsmittel realisiert wird und einen Schritt des erneuten Abtastens der RR-Reihe aufweist, um eine erneut abgetastete RR-Reihe zu erhalten,
**dadurch gekennzeichnet, dass**

das Verfahren weiterhin einen Schritt zur automatischen Kontrolle der Qualität der R-Reihe durch automatisches Berechnen mindestens des mathematischen Normwerts (NORME), innerhalb eines gleitenden Fensters, der erneut abgetasteten RR-Reihe beinhaltet, wobei der mathematische Normwert durch die folgende Formel angegeben wird:

$$NORME \;=\; \sqrt{\sum_{i=1}^{N}\left(RR_i - \frac{1}{N}\sum_{i=1}^{N}\left(RR_i\right)\right)^2}$$

wobei N die Anzahl der Abtastungen $RR_i$ in diesem Fenster ist.

2. Verfahren nach Anspruch 1, bei dem in der anfänglichen RR-Reihe automatisch erkannt wird, ob eine oder mehrere aufeinanderfolgende Abtastungen (RRi) fehlerhaft sind, und die als fehlerhaft erkannte(n) Abtastung(en) (RRi) in der RR-Reihe automatisch korrigiert wird (werden), indem sie durch eine oder mehrere rekonstruierte Abtastungen (RRc) ersetzt wird (werden), und wobei das erneute Abtasten an der gegebenenfalls teilweise rekonstruierten RR-Reihe durchgeführt wird, um eine gegebenenfalls teilweise rekonstruierte und erneut abgetastete RR-Reihe zu erhalten.

3. Verfahren nach einem der vorhergehenden Ansprüche, bei dem automatisch eine Aktion ausgelöst wird, wenn der berechnete mathematische Normwert (NORME) unter einem vordefinierten Wert (NormMin) liegt, oder wenn der berechnete mathematische Normwert (NORME) über einem vordefinierten Wert (NormMax) liegt, oder wenn der berechnete mathematische Normwert (NORME) unter einem vordefinierten Wert (NormMax) liegt, oder wenn der berechnete mathematische Normwert (NORME) außerhalb eines vorgegebenen Bereichs liegt (NormMin; Norm-Max).

4. Verfahren nach einem der vorhergehenden Ansprüche, bei dem die Qualität der RR-Reihe automatisch kontrolliert wird, indem auch die momentane Herzfrequenz $FC_i$ berechnet wird, wobei $FC_i = 60000/RR_i$, wobei $RR_i$ der momentane Millisekundenwert einer Abtastung (RRi) aus der RR-Reihe ist.

5. Verfahren nach Anspruch 4, bei dem automatisch eine Aktion ausgelöst wird, wenn die berechnete momentane Herzfrequenz FCi unterhalb eines vorgegebenen Wertes (FCMin) liegt.

6. Verfahren nach Anspruch 4, bei dem automatisch eine Aktion ausgelöst wird, wenn die berechnete momentane Herzfrequenz FCi einen vorgegebenen Wert (FCMax) übersteigt.

7. Verfahren nach Anspruch 4, bei dem automatisch eine Aktion ausgelöst wird, wenn die berechnete momentane Herzfrequenz FCi außerhalb eines vorgegebenen Bereichs (FCMin; FCMax) liegt.

8. Verfahren nach einem der Ansprüche 3, 5, 6, 7, wobei die Aktion, die ausgelöst wird, das Auslösen eines visuellen und/oder akustischen Alarms umfasst.

9. Verfahren nach einem der Ansprüche 3, 5, 6, 7, wobei die Aktion, die ausgelöst wird, das Zurücksetzen der Erfassung und Konstruktion der Abtastungen (RRi) der anfänglichen RR-Reihe umfasst.

10. Verfahren nach einem der vorhergehenden Ansprüche, wobei ein Qualitätsindex (NivQual) zumindest aus dem berechneten mathematischen Normwert (NORME) berechnet wird.

11. Verfahren nach einem der vorhergehenden Ansprüche, wobei mindestens ein Qualitätsindex (NivQual) aus der momentanen Herzfrequenz $FC_i$ berechnet wird, wobei $FC_i = 60000/RR_i$, wobei $RR_i$ der momentanen Millisekundenwert einer Abtastung (RRi) der RR-Reihe ist.

12. Verfahren nach einem der vorhergehenden Ansprüche, welches in Echtzeit die Erfassung und Konstruktion aufeinanderfolgender ($RR_i$)-Abtastungen der anfänglichen RR-Reihe aus einem Herzsignal beinhaltet, wobei das erneute Abtasten und die automatische Qualitätskontrolle der RR-Reihe in Echtzeit entsprechend der Erfassung und Konstruktion der aufeinanderfolgenden Abtastungen (RRi) der anfänglichen RR-Reihe durchgeführt werden.

13. Vorrichtung (3) zur Verarbeitung einer RR-Reihe, die aus einer Vielzahl von Abtastungen (RRi) besteht, die jeweils eine Funktion von zwei aufeinanderfolgende Herzschläge trennenden Zeitintervallen (δti) sind, wobei die Vorrichtung (3) so ausgelegt ist, dass sie automatisch die Qualität der RR-Reihe kontrolliert, indem sie das Verfahren gemäß einem der vorhergehenden Ansprüche durchführt.

14. System zur Erfassung und Verarbeitung eines Herzsignals, wobei das System elektronische Mittel (1, 2) zur Erfassung eines Herzsignals und elektronische Verarbeitungsmittel (3) umfasst, die so ausgelegt sind, dass sie aus dem von den elektronischen Erfassungsmitteln (1, 2) erfassten Herzsignal eine anfängliche RR-Reihe bilden/konstruieren, wobei die RR-Reihe aus einer Vielzahl von Abtastungen (RRi) gebildet ist, die jeweils eine Funktion von zwei aufeinanderfolgende Herzschläge trennenden Zeitintervallen (δti) sind, wobei die elektronischen Verarbeitungsmittel (3) so ausgelegt sind, dass sie die Qualität der RR-Reihe durch Durchführung des Verfahrens nach einem der Ansprüche 1 bis 12 automatisch kontrollieren.

15. Computerprogramm mit einem Computerprogrammcode, das/der durch elektronische Verarbeitungsmittel (3) ausgeführt werden kann und bei Ausführung durch elektronische Verarbeitungsmittel (3) die Durchführung des Verfahrens zur Kontrolle der Qualität einer RR-Reihe nach einem der Ansprüche 1 bis 12 ermöglicht.

**Claims**

1. A method for controlling the quality of an initial RR series consisting of a plurality of samples ($RR_i$) which are respectively a function of time intervals (δti) which separate two successive heartbeats, method implemented by electronic processing means and comprising a step of re-sampling of the RR series so as to obtain a resampled RR series, **characterized in** said method further comprises a step of automatic control of the quality of the RR series by automatically calculating at least the mathematical norm value (NORME), in a sliding window, of the resampled RR series, said mathematical norm value being given by the following formula:

$$NORME = \sqrt{\sum_{i=1}^{N}\left(RR_i - \frac{1}{N}\sum_{i=1}^{N}(RR_i)\right)^2}$$

where N is the number of $RR_i$ samples in said window.

2. The method according to Claim 1, during which one automatically detects in the initial RR series if one or more successive (RRi) samples are incorrect, and automatically corrects, in the RR series, the one or more (RRi) samples detected as incorrect by replacing them by one or more reconstructed (RRc) samples, so as to obtain an RR series optionally partly reconstructed, and wherein the resampling is performed on the RR series, optionally partly reconstructed, so as to obtain an optionally partly reconstructed and resampled RR series.

3. Method according to any of the preceding claims, wherein one automatically triggers an action when the mathematical norm value (NORME) that is calculated is smaller than a predefined value (NormMin) or when the mathematical norm value (NORME) that is calculated is greater than a predefined value (NormMax) or when the mathematical norm value that is calculated is outside a predefined range (NormMin; NormMax).

4. Method according to any one of the preceding claims, wherein one automatically controls the quality of the RR series by also calculating the instantaneous heart rate $FC_i$, where $FC_i = 60000/RR_i$, $RR_i$ being the instantaneous value in millisecond of an ($RR_i$) sample of the RR series.

5. The method according to Claim 4, wherein one automatically triggers an action when the instantaneous heart rate $FC_i$ that is calculated is lower than a predefined value (FCMin).

6. The method according to Claim 4, wherein one automatically triggers an action when the instantaneous heart rate $FC_i$ that is calculated is greater than a predefined value (FCMax).

7. The method according to Claim 4, wherein one automatically triggers an action when the instantaneous heart rate $FC_i$ that is calculated is outside of a predefined range (FCMin, FCMax).

8. The method according to any one of Claims 3, 5, 6, 7, wherein the action that is triggered comprises the triggering of a visual and/or audible alarm.

9. The method according to any one of Claims 3, 5, 6, 7, wherein the action that is triggered comprises resetting the acquisition and construction of the ($RR_i$) samples of the initial RR series.

10. Method according to any of the preceding claims, wherein one calculates a (NivQual) quality index from at least the mathematical norm value (NORME) that is calculated.

11. The method according to any of the preceding claims, wherein one calculates at least one (NivQual) quality index from the instantaneous heart rate $FC_i$, with $FC_i = 60000 / RR_i$, $RR_i$ being the instantaneous value in millisecond of a ($RR_i$) sample of the RR series.

12. Method according to any of the preceding claims, comprising the acquisition and construction in real time of successive ($RR_i$) samples of the initial RR series from a cardiac signal, and wherein the resampling and the automatic quality control of the RR series are performed in real time while said acquisition and construction of the successive ($RR_i$) samples of the initial RR series are taking place.

13. Processing device (3) of an RR series consisting of a plurality of ($RR_i$) samples which are respectively a function of time intervals ($\delta t_i$) separating two successive heartbeats, said device (3) being arranged so as to automatically control the quality of the RR series by implementing the method described in any of the preceding claims.

14. Acquisition and processing system for a cardiac signal, said system comprising electronic acquisition means (1,2) of a cardiac signal, and electronic processing means (3) arranged so as to construct an initial RR series from the cardiac signal acquired by the electronic acquisition means (1,2), said RR series consisting of a plurality of ($RR_i$) samples which are respectively a function of time intervals ($\delta t_i$) which separate two successive heartbeats of the cardiac signal, said electronic processing means (3) being arranged so as to automatically control the quality of this RR series by implementing the method referred to in any one of Claims 1 to 12.

15. A computer program comprising coding means for a computer program adapted to be executed by electronic processing means (3), and allowing, when executed by electronic processing means (3), to implement the quality control method of an RR series referred to in any one of Claims 1 to 12.

FIG.1

FIG.2

FIG. 3

FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02069178 A

**Littérature non-brevet citée dans la description**

- **LACROIX D ; LOGIER R. ; KACET S. ; HAZARD J-R ; DAGANO J.** *Effects of consecutive administration of central and peripheral anticholinergic agents on respiratory sinu arrhytmia in normal subjects, J. of the Autonomie Nervous System,* 1992, vol. 39, 211-218

- **LOGIER R ; DE JONCKHEERE J ; DASSONNEVILLE A.** An efficient algorithm for R-R intervals series filtering. *Conf Proc IEEE Eng Med Biol Soc.,* 2004, vol. 6, 3937-40
- **VARADARAJAN V et al.** Assessing QT-RR interval hystérésis in 12-lead electrocardiograms. *COMPUTERS IN CARDIOLOGY, 2009, IEEE, PISCATAWAY, NJ, USA,* 13 Septembre 2009, 273-276